# FASCICULE DE BREVET EUROPEEN

(11) **EP 4 551 293 B1**
(45) Date de publication et mention de la délivrance du brevet: **05.08.2026**
(21) Numéro de dépôt: 23736171.2
(22) Date de dépôt: 09.06.2023
(51) Int. Cl.: A61N 5/06

(54) **TABLETTE DE PHOTOTHÉRAPIE DÉFORMABLE**
VERFORMBARE PHOTOTHERAPIETABLETTE
DEFORMABLE PHOTOTHERAPY TABLET

(30) Priorité: 04.07.2022 FR 2206739; 29.12.2022 FR 2214672
(43) Date de publication de la demande: 14.05.2025
(73) Titulaire: Lucibel SA, 76360 Barentin (FR)
(72) Inventeur: DAVID, Thibault, 76240 BONSECOURS (FR); GROS-DAILLON, Thibault, 76590 CRIQUETOT-SUR-LONGUEVILLE (FR)
(74) Mandataire: Martin, Marie-Aude
(86) Numéro de dépôt international: PCT/FR2023/050838
(87) Numéro de publication internationale: WO 2024/009017

(56) Documents cités:
- WO-A1-01/14012
- CN-A- 108 837 315
- FR-A1- 3 034 022
- US-A1- 2004 223 328
- US-A1- 2021 108 793

## Description

La présente invention concerne un dispositif de photothérapie, pour le traitement d'une région externe du corps d'un être vivant à usage médical ou cosmétique. Elle s'applique plus particulièrement mais non exclusivement à une utilisation au contact intime de la peau d'un utilisateur.

Le traitement par photothérapie, par exemple divulguée dans le document FR 3 034 022 A1, consiste en l'application d'un rayonnement lumineux présentant un spectre d'émission prédéfini directement sur la région du corps à traiter. Le rayonnement est de préférence sensiblement monochromatique ou présente un spectre d'émission compris dans une bande étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules, mais également certains cancers cutanés.

L'invention s'applique plus particulièrement aux dispositifs de photothérapie non rigides et pouvant se déformer au moins légèrement sous l'effet d'une pression manuelle.

De tels dispositifs permettent d'améliorer l'efficacité de pénétration de la lumière de photothérapie à l'intérieur des tissus du corps du patient en s'adaptant à la forme de la région du corps à traiter, que ce soit le visage, la main, le buste, etc. Cette conformation permet de limiter la présence de zones d'ombre sur le visage.

Il est par ailleurs aisé dans ce cas de maintenir le dispositif contre la région du corps à traiter grâce à différents moyens d'attache qui laissent les mains libres à l'utilisateur. L'effet produit sur l'utilisateur un état de relaxation accru et un plus grand bien-être.

On connaît déjà de l'art antérieur un dispositif de photothérapie à diodes électroluminescentes se présentant sous forme d'un bandage. Dans ce cas, la source lumineuse à diodes électroluminescentes est portée par une plaque de circuit imprimé incorporée à l'intérieur du bandage. La plaque de circuit imprimé présente des découpages prédéfinis permettant une flexion améliorée de la plaque.

L'inconvénient d'un tel dispositif est que généralement la plaque de circuit imprimé a de très faibles propriétés élastiques et qu'au bout d'un certain nombre de flexions du dispositif, la plaque peut présenter des zones de fragilisation, voire des zones de rupture pouvant conduire à des dysfonctionnements électroniques du dispositif.

Un autre inconvénient des dispositifs de l'art antérieur est de présenter un risque de surchauffe qui peut produire un inconfort chez l'utilisateur, voire parfois des brûlures sur la peau.

L'invention a notamment pour but de remédier à ces inconvénients en proposant un dispositif de photothérapie permettant de se déformer tout en préservant l'intégrité de la source lumineuse et de son circuit électronique en assurant une dissipation thermique.

### Description de l'invention

A cet effet, l'invention a pour objet un dispositif lumineux souple et déformable, en particulier adapté pour venir en contact sur une surface à traiter de la peau d'un utilisateur, du type comprenant une source lumineuse comprenant une pluralité de composants d'émission lumineuse et un réseau d'interconnexion électrique des composants entre eux pour leur alimentation électrique, une couche de dissipation thermique de la chaleur émise par la source lumineuse, un substrat déformable délimitant une face d'émission du flux lumineux, incorporant dans sa masse, la source lumineuse, caractérisé en ce que la couche de dissipation thermique est formée par une juxtaposition de plaquettes déformables métalliques électriquement et thermiquement conductrices, les plaquettes étant disjointes et séparées par des interstices formant un réseau inverse de pistes d'isolation électrique des plaquettes métalliques entre elles pour définir par complémentarité le réseau d'interconnexion électrique des composants entre eux dans la couche de dissipation thermique.

Grâce à la surface d'échange thermique augmentée de la tablette de l'invention, le dispositif de photothérapie permet une dissipation thermique efficace de la chaleur émise par les composants lumineux. Ceci permet d'assurer à l'utilisateur une sensation agréable de chaleur sans inconfort lié à une surchauffe des composants. Ceci permet également d'offrir à l'utilisateur une perception de grande qualité du dispositif tout en formant un ensemble uniformément esthétique du dispositif.

Le principe de l'invention repose en effet sur une optimisation de la surface occupée par les connecteurs électriques du réseau d'interconnexion électrique qui forment eux-mêmes des dissipateurs thermiques très efficaces.

Le dispositif selon l'invention peut en outre comprendre l'une ou plusieurs des caractéristiques suivantes.

Selon un mode de réalisation préféré de l'invention, chaque composant est monté en pont entre deux plaquettes.

Selon un mode de réalisation préféré de l'invention, les plaquettes déformables métalliques forment des languettes souples formant une matrice de bandes sensiblement parallèles, juxtaposées et séparées les unes des autres par les interstices et les composants sont montés en pont entre deux plaquettes adjacentes en formant une rangée de composants connectés électriquement en parallèle par les deux plaquettes adjacentes.

Selon un mode de réalisation préféré de l'invention, le dispositif comprend des première et deuxième sources lumineuses et des première et deuxième couches de dissipation thermique de telle sorte que le dispositif est à double face d'émission lumineuse.

Selon un mode de réalisation préféré de l'invention, le composant est une diode électroluminescente.

Selon un mode de réalisation préféré de l'invention, le dispositif comprend au moins une plaquette latérale d'angle avec une forme générale en L comprenant une borne d'extrémité de contact formant plot de contact pour le raccordement électrique à un connecteur d'alimentation électrique externe.

Selon un mode de réalisation préféré de l'invention, la plaquette métallique est réalisée dans un matériau comprenant essentiellement du cuivre.

Selon un mode de réalisation préféré de l'invention, la plaquette a une forme générale polygonale, par exemple rectangulaire, carrée ou en « L », et/ou à bords arrondis.

Selon un mode de réalisation préféré de l'invention, la couche du substrat est réalisée dans un matériau comprenant essentiellement du polyuréthane.

Selon un mode de réalisation préféré de l'invention, chaque plaquette est formée d'un feuillet de cuivre.

Selon un mode de réalisation préféré de l'invention, l'épaisseur de chaque feuillet de cuivre est supérieure à 30 micromètres, de préférence supérieure à 100 micromètres.

Selon un mode de réalisation préféré de l'invention, le dispositif comprend une couche intermédiaire de renforcement.

Selon un mode de réalisation préféré de l'invention, la couche intermédiaire comprend un PET transparent.

Selon un mode de réalisation préféré de l'invention, les composants lumineux émettent dans un spectre rouge de longueur d'onde comprise entre 620 et 640 nm.

Selon un mode de réalisation préféré de l'invention, la surface du réseau inverse de pistes d'isolation électrique représente moins de 10% de la surface de la couche de dissipation thermique et de préférence moins de 5%, et au mieux de préférence moins de 2%.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig. 1] La figure 1 est une vue en perspective d'un dispositif de photothérapie selon l'invention ;
[Fig. 2] La figure 2 est une vue schématique de dessus d'une source lumineuse du dispositif selon l'invention ;
[Fig. 3] La figure 3 est une vue schématique en coupe transversale du dispositif de photothérapie de la figure 1 ;
[Fig. 4] La figure 4 représente une vue de dessus (4A), de dessous (4B), de côté (4C), en perspective (4D) d'un composant lumineux de la source lumineuse du dispositif de la figure 1 ;

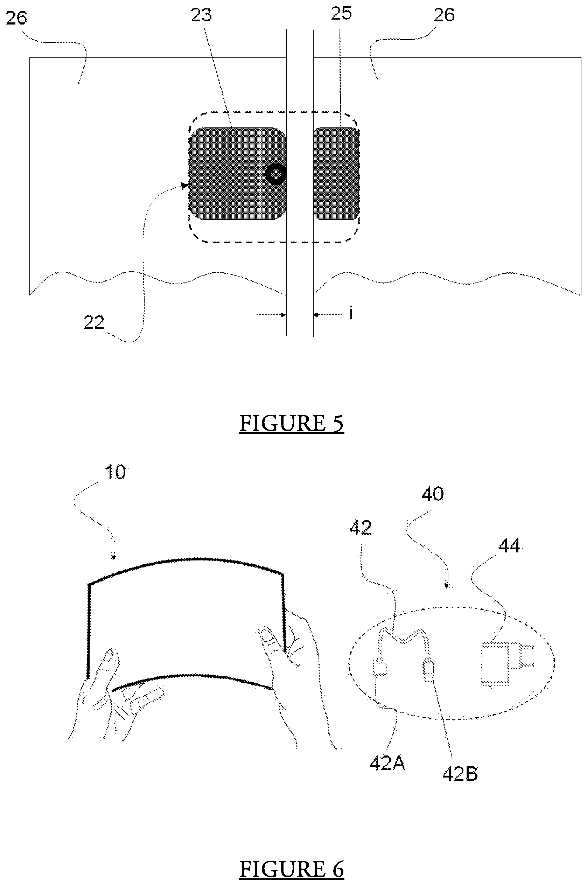

[Fig. 5] La figure 5 est une vue partielle à échelle agrandie du composant lumineux de la figure 4 ;
[Fig. 6] La figure 6 est une vue en perspective du dispositif de photothérapie dans une configuration déformée par un utilisateur ;
[Fig. 7] La figure 7 représente un exemple d'application du dispositif de photothérapie.
[Fig. 8] La figure 8 est une vue schématique en coupe transversale d'une variante du dispositif de photothérapie de la figure 1.

### Description détaillée de l'invention

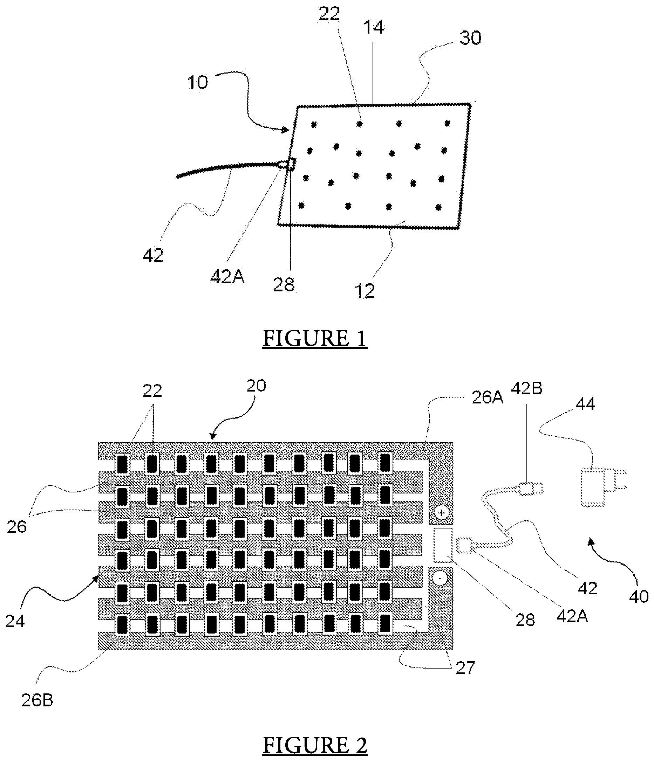

On a représenté de façon schématique sur la figure 1 un dispositif de photothérapie selon l'invention. Le dispositif est désigné par la référence générale 10.

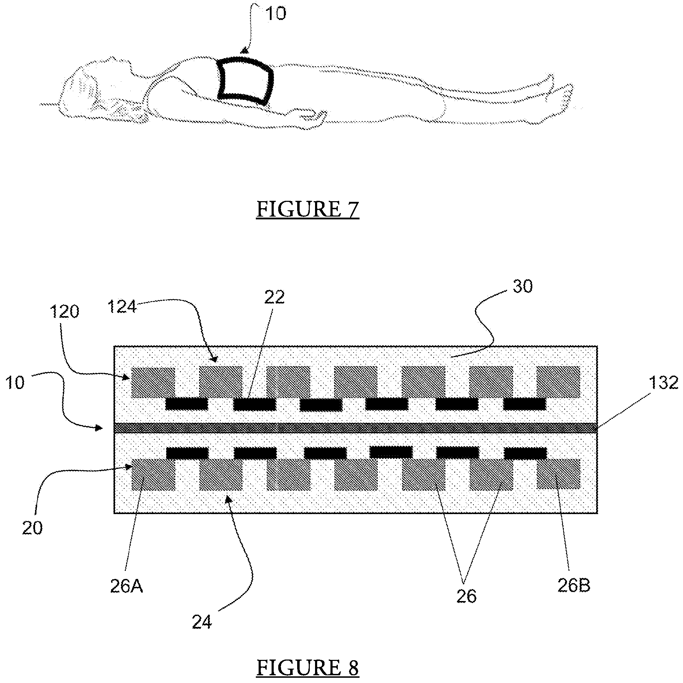

Dans l'exemple illustré, le dispositif 10 se présente sous la forme d'une tablette souple et déformable adaptée pour venir en contact sur une surface à traiter de la peau d'un utilisateur (figure 7).

La tablette 10 comprend une source lumineuse 20 comprenant une pluralité de composants 22 d'émission lumineuse et un réseau 24 d'interconnexion électrique des composants 22 entre eux pour leur alimentation électrique.

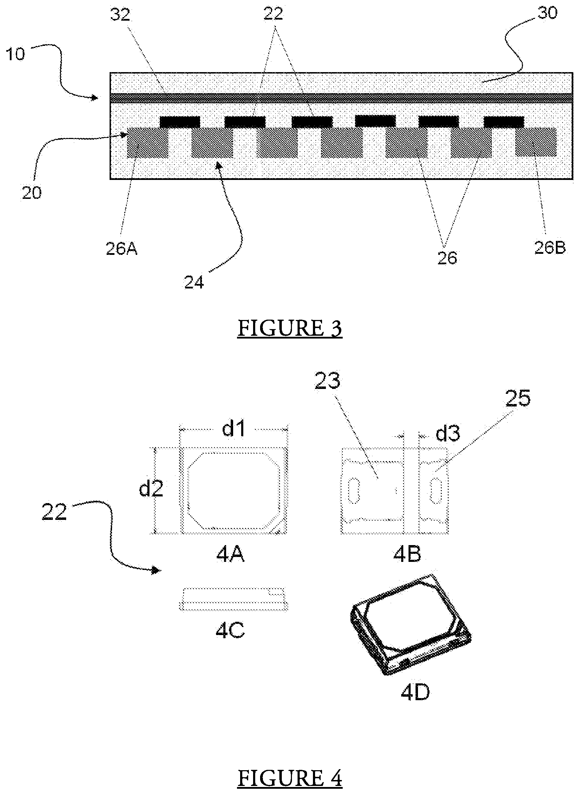

Comme illustré sur la figure 3, la tablette 10 est formé par un substrat 30 déformable délimitant une face 12 d'émission du flux lumineux et une face 14 opposée de présentation. Le substrat 30 incorpore totalement dans sa masse la source lumineuse 20. La couche du substrat 30 est réalisée par exemple dans un matériau comprenant essentiellement du polyuréthane. Le matériau se présente sous la forme d'une résine d'enrobage ou d'encapsulation de la source lumineuse 20 qui permet d'isoler et de protéger le réseau d'interconnexion électrique 24 et les composants 22 des agressions inhérentes à des environnements difficiles (humidité, chocs thermiques ou physiques et les contaminants en général (sueur, poussière, résidus de produits cosmétiques, etc...)).

Le matériau du substrat se présente avant transformation par exemple sous forme d'une masse pouvant s'écouler dans un moule délimitant une cavité libre de moulage dimensionnée au format de la tablette 10 souhaitée à l'intérieur de laquelle est positionnée la source lumineuse 60. Puis le matériau du substrat 30 subit une étape de durcissement par divers procédés qui sont connus de l'homme du métier.

De préférence, les composants d'émission lumineuse 22 comprennent des diodes électroluminescentes. Par exemple, les composants lumineux 22 émettent dans un spectre rouge de longueur d'onde comprise entre 620 et 640 nm, de préférence autour de 630 nanomètres.

Un tel composant d'émission lumineuse 22 est représenté en détail sur la figure 4. Dans l'exemple illustré, ce composant comprend une face de dessus d'émission lumineuse et une face de dessous de montage sur un support pour son raccordement électrique. La face de dessous comprend dans l'exemple décrit deux emplacements délimitant des pastilles de soudage pour le montage du composant sur un support tel que cela sera décrit ci-après.

Le composant lumineux 22 est dans cet exemple une diode électroluminescente émettant principalement dans une plage comprise entre 620 et 640 nanomètres, de préférence autour de la longueur d'onde de 630 nanomètres. Par exemple, la diode électroluminescente 22 est une diode de haute puissance avec une tension nominale d'environ 3 Volts. Chaque diode est par exemple commandée par le circuit de pilotage avec un courant de l'ordre de 120 mA sous une tension de 3.3 Volts, ce qui représente une puissance d'environ 400 mW environ. Par ailleurs, de préférence, la puissance lumineuse de chaque diode 22 est supérieure à 5 lumens, de préférence supérieures à 15 lumens.

Dans l'exemple décrit, le composant lumineux 22 a une forme générale de boîtier avec une largeur de 1.90 millimètres et une longueur de 2.03 millimètres.

Selon l'invention, la tablette 10 comprend une couche 24 de dissipation thermique de la chaleur émise par la source lumineuse 20. Conformément à l'invention, la couche de dissipation thermique est formée par une juxtaposition de plaquettes déformables métalliques 26 électriquement et thermiquement conductrices.

Comme cela apparaît clairement sur la figure 2, les plaquettes 26 sont disjointes et séparées par des interstices 27 formant des pistes d'isolation électrique des plaquettes métalliques 26 entre elles. Par « interstice », on entend au sens de la présente invention, un petit espace vide entre les parties d'un tout formé, dans le cas présent, par les plaquettes 26.

En particulier, les plaquettes 26 sont disjointes et séparées par les interstices 27 formant un réseau inverse de pistes d'isolation électrique des plaquettes métalliques 26 entre elles ce qui permet de définir des chemins de conduction électrique délimitant par complémentarité le réseau d'interconnexion électrique 24 des composants 22 entre eux dans la couche de dissipation thermique.

On comprend donc de l'invention, que les plaquettes 26 sont juxtaposées à la manière d'une surface dallée ou carrelée pour former un circuit inversé de pistes d'isolation électrique. Ceci permet d'occuper une surface maximale de la tablette 10 tout en assurant une fonction de réseau d'interconnexion électrique des composants entre eux grâce à la présence des pistes électriquement isolantes qui définissent et délimitent les chemins de conductions électriques du réseau d'interconnexion électrique 24.

Ainsi, l'ensemble des plaquettes 26 forme une surface d'échange thermique augmentée permettant une action de dissipation de la chaleur des composants 22 vers l'extérieur de la tablette 10.

On peut comprendre que la largeur des interstices est définie de telle sorte que les plaquettes 26 soient isolées électriquement les unes des autres. De préférence, la largeur des interstices est d'environ 0.4 à 1 millimètre, de préférence autour de 0.5 millimètres.

De préférence, chaque composant 22 est monté en pont entre deux plaquettes 26. Par exemple, les plaquettes déformables métalliques 26 forment des languettes souples formant une pluralité de bandes sensiblement parallèles, juxtaposées et séparées les unes des autres par les interstices 27 et les composants 22 sont montés en pont entre deux plaquettes adjacentes 26 en formant une rangée de composants 22 connectés électriquement en parallèle par les deux plaquettes 26.

On a illustré en détail un tel montage du composant 22 en pont sur deux plaquettes 26. On voit que le composant comprend une première pastille de soudage 23 s'étendant sur une première plaquette et une deuxième pastille de soudage 25 s'étendant sur une deuxième plaquette adjacente, les deux pastilles étant séparées d'un interstice d'environ 0.5 millimètres. Dans l'exemple décrit, l'interstice i est compris dans une plage de valeurs de 0.4 à 1 exprimée en millimètres. La première pastille 23 forme dans cet exemple l'anode de la diode 22.

En outre, comme cela apparaît clairement sur la figure 3, le réseau 24 comprend au moins une plaquette latérale d'angle 26A, 26B avec une forme générale en L comprenant une borne d'extrémité de contact formant plot de contact pour le raccordement électrique à un port de connecteur 28 d'alimentation électrique du dispositif 10.

De préférence, les plaquettes 26 ont une forme générale polygonale, par exemple rectangulaire, carrée ou en « L », et/ou à bords arrondis.

De préférence, chaque plaquette métallique 26 est réalisée dans un matériau comprenant essentiellement du cuivre et par exemple se présentant sous forme d'un feuillet de cuivre. Par exemple, l'épaisseur de chaque feuillet de cuivre est supérieure à 30 micromètres, de préférence supérieure à 100 micromètres afin d'assurer une dissipation thermique efficace.

Dans le mode illustré par les figures, le réseau 22 présente une topologie sous forme matricielle de rangées de plaquettes 26 qui couvre une surface maximale de la tablette 10.

La proportion de la surface des plaquettes (ce qui correspond à la surface du réseau d'interconnexion électrique 24) par rapport à la surface totale des réseaux inverse et de connexion est de préférence supérieure à 90%, de préférence supérieure à 95% et dans cet exemple autour de 98%. Dans le cadre de la présente invention, le terme « autour de » est synonyme de « environ » et signifie plus ou moins 10% de la valeur nominale de ce nombre.

Autrement dit, la surface du réseau inverse de pistes 27 d'isolation électrique représente moins de 10% de la surface de la couche de dissipation thermique 24 et de préférence moins de 5%, et au mieux de préférence moins de 2%.

Dans l'exemple illustré, les bandes ont une longueur d'environ 30 millimètres, une largeur d'environ 5 à 10 millimètres et les interstices ont une largeur d'environ 0.4 à 1 millimètre.

Le positionnement des plaquettes 26 est optimisée afin de couvrir une surface maximale de la tablette.

Comme cela est illustré sur la figure 3, les plaquettes déformables métalliques 26 forment des languettes souples formant une pluralité de bandes sensiblement parallèles, juxtaposées et séparées les unes des autres par les interstices 27. Ainsi, dans ce cas, les composants 22 sont montés en pont entre deux plaquettes adjacentes 26, 26A, 26B en formant une rangée de composants 22 connectés électriquement en parallèle par les deux plaquettes 26.

Par exemple, chaque plaquette métallique 22 est réalisée dans un matériau comprenant essentiellement du cuivre. La plaquette 30 a une forme générale polygonale, par exemple rectangulaire, carrée ou en « L », et/ou à bords arrondis.

De préférence, le substrat 30 comprend une couche intermédiaire de renforcement 32 configurée pour imposer une déformation continue et contrôlée au substrat 30.

De préférence, la couche intermédiaire 32 a une épaisseur comprise entre 400 micromètres et 600 micromètres. Par exemple, la couche intermédiaire 32 comprend un PET (polyéthylène téréphtalate) transparent.

Grâce à la présence de cette couche intermédiaire 32 de renforcement, lorsque le dispositif 10 est soumis à des forces de déformations, la couche intermédiaire de renforcement 32 permet d'imposer une déformation continue et contrôlée du substrat 30 afin d'éviter de fortes contraintes de cisaillement et/ou de pliures qui peuvent fragiliser localement et entraîner par exemple des ruptures de continuité électrique entre un composant 22 et une plaquette 26.

De préférence, les plaquettes 26 ont une forme générale rectangulaire comme cela est illustré sur la figure 3. Toutefois, l'invention ne se limite pas à une forme particulière des plaquettes 26 et peut prendre toute autre forme ou dimensions. Par exemple, dans une variante non illustrée, les plaquettes 26 peuvent avoir une forme générale carrée.

Par ailleurs, comme cela est illustré sur la figure 1, la tablette 10 est, dans cet exemple, pourvu du port connecteur 28 qui est agencé en périphérie de la tablette 10 afin de présenter une interface d'alimentation électrique avec des moyens de raccordement externes 40. Par exemple, cette interface peut être du type à couplage magnétique encliquetable avec une prise connecteur complémentaire 42A des moyens de raccordement 40 pour l'alimentation électrique de la source lumineuse 20.

La prise 42A et le port 28 peuvent présenter une symétrie de rotation de telle sorte qu'une rotation à 180 degrés de l'un ou l'autre de la prise ou du port, dans le sens horaire ou antihoraire, entraîne une connexion électrique identique, éliminant ainsi la nécessité de vérifier l'alignement lors de l'établissement d'une connexion. En variante, la prise 42A et le port 28 ne peuvent être raccordés que dans une unique position. Dans ce cas, les éléments aimantés de l'interface à couplage magnétique sont polarisés de telle manière que l'utilisateur ne peut connecter l'interface que dans une unique position. Dans cette variante, l'interface est ainsi munie d'un détrompeur de sorte qu'en cas d'inversion du sens relatif de la prise 42A et du port 28, les éléments aimantés se repoussent.

Dans l'exemple décrit, le port connecteur 28 permet de former une interface connectable accessible depuis l'extérieur de la tablette 10 pour la connexion de la prise connecteur 42A des moyens de raccordement 40, complémentaire au port connecteur 28. De préférence, cette interface connectable est du type à couplage magnétique et encliquetage.

Par ailleurs, la prise connecteur 42A est reliée également à une première extrémité d'un câble d'alimentation électrique 42, ce câble 42 étant pourvu à son autre extrémité d'un connecteur 42B de type « USB » ou équivalent. A cet effet, de préférence, les moyens de raccordement 40 comprennent un bloc prise connecteur séparable 44, compatible « USB » de format standard pour le raccordement électrique à un réseau d'alimentation électrique domestique.

On a illustré sur la figure 7, une application particulière du dispositif de photothérapie 10 pour une utilisation sur une portion ventrale du corps d'un utilisateur. La tablette souple et déformable 10 est disposée contre la surface du corps à traiter de l'utilisateur.

Dans la variante illustrée sur la figure 8, la tablette 10 est à double face d'émission lumineuse et comprend à cet effet des première et deuxième sources lumineuses 20 et 120 comprenant chacune une pluralité de composants 22 d'émission lumineuse et des premier et deuxième réseaux 24 et 124 d'interconnexion électrique des composants 22 entre eux pour leur alimentation électrique.

Comme décrit précédemment pour le cas d'une tablette à une seule face d'émission lumineuse, chaque couche 24, 124 de dissipation thermique est formée par une juxtaposition de plaquettes 26, 26A, 26B déformables métalliques électriquement et thermiquement conductrices, les plaquettes 26, 26A, 26B étant disjointes et séparées par des interstices 27.

En particulier, les interstices 27 de chaque couche 24 ou 124 forment un réseau inverse de pistes d'isolation électrique des plaquettes métalliques 26, 26A, 26B entre elles pour définir par complémentarité le réseau d'interconnexion électrique 24 ou 124 des composants 22 entre eux dans la couche de dissipation thermique 24 ou 124.

Dans cette variante, la tablette 10 est formé par un substrat 30 déformable délimitant des faces opposées d'émission du flux lumineux. La tablette 10 est une tablette double face et émet de la lumière par chacune de ses faces opposées. De préférence, comme cela apparaît sur la figure 8, le substrat 30 comprend une couche intermédiaire 132 d'isolation thermique qui sépare thermiquement les deux réseaux d'interconnexion électrique 24 et 124. En outre, avantageusement, cette couche intermédiaire 132, réalisée par exemple en PET (polyéthylène téréphtalate) permet de renforcer la tablette 10 et est configurée pour imposer une déformation continue et contrôlée au substrat 30.

Bien que non illustrée, les deux réseaux 24 et 124 sont raccordés électriquement entre eux de sorte que la tablette 10 ne comporte qu'un unique port connecteur 28 pour l'alimentation des deux sources lumineuses 20 et 120.

Une application particulière de cette tablette conforme à la variante illustrée sur la figure 8 peut être prévue pour une utilisation dans l'entrecuisse en positionnant la tablette double face verticalement entre les jambes. Ceci permet d'agir simultanément sur les parties corporelles des jambes qui se font face.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple. Par exemple, d'autres applications de la tablette peuvent être envisagées sans sortir de cadre de l'invention, par exemple une application pour la stimulation de la la croissance ou de la photosynthèse d'organismes vivants.

## Revendications

1. Dispositif (10) lumineux souple et déformable, en particulier adapté pour venir en contact sur une surface à traiter de la peau d'un utilisateur, du type comprenant :
- une source lumineuse (20, 120) comprenant une pluralité de composants (22) d'émission lumineuse et un réseau (24, 124) d'interconnexion électrique des composants (22) entre eux pour leur alimentation électrique,
- une couche (24, 124) de dissipation thermique de la chaleur émise par la source lumineuse (20),
- un substrat (30) déformable délimitant une face d'émission du flux lumineux, incorporant dans sa masse, la source lumineuse (20), la couche (24, 124) de dissipation thermique étant formée par une juxtaposition de plaquettes (26, 26A, 26B) déformables métalliques électriquement et thermiquement conductrices, les plaquettes (26, 26A, 26B) étant disjointes et séparées par des interstices (27) formant un réseau inverse de pistes d'isolation électrique des plaquettes métalliques (26, 26A, 26B) entre elles pour définir par complémentarité le réseau d'interconnexion électrique (24, 124) des composants (22) entre eux dans la couche de dissipation thermique (24, 124).

2. Dispositif (10) selon la revendication précédente, comprenant des première (20) et deuxième (120) sources lumineuses et des première (24) et deuxième (124) couches de dissipation thermique de telle sorte que le dispositif (10) est à double face d'émission lumineuse.

3. Dispositif (10) selon l'une quelconque des revendications précédentes, dans lequel chaque composant (22) est monté en pont entre deux plaquettes (26, 26A, 26B).

4. Dispositif (10) selon l'une quelconque des revendications précédentes, dans lequel les plaquettes déformables métalliques (26, 26A, 26B) forment des languettes souples formant une matrice de bandes sensiblement parallèles, juxtaposées et séparées les unes des autres par les interstices (27) et les composants (22) sont montés en pont entre deux plaquettes adjacentes (26, 26A, 26B) en formant une rangée de composants (22) connectés électriquement en parallèle par les deux plaquettes adjacentes (26, 26A, 26B).

5. Dispositif (10) selon l'une quelconque des revendications précédentes, dans lequel le composant (22) est une diode électroluminescente.

6. Dispositif (10) selon l'une quelconque des revendications précédentes, comprenant au moins une plaquette latérale d'angle (26A, 26B) avec une forme générale en L comprenant une borne d'extrémité de contact formant plot de contact pour le raccordement électrique à un connecteur (28) d'alimentation électrique externe.

7. Dispositif (10) selon l'une quelconque des revendications précédentes, dans lequel la plaquette métallique (26) est réalisée dans un matériau comprenant essentiellement du cuivre.

8. Dispositif (10) selon la revendication précédente, dans lequel la plaquette (26) a une forme générale polygonale, par exemple rectangulaire, carrée ou en « L », et/ou à bords arrondis.

9. Dispositif (10) selon l'une quelconque des revendications précédentes, dans lequel la couche du substrat (30) est réalisée dans un matériau comprenant essentiellement du polyuréthane.

10. Dispositif (10) selon l'une quelconque des revendications précédentes, dans lequel chaque plaquette (26) est formé d'un feuillet de cuivre.

11. Dispositif (10) selon la revendication précédente, dans lequel l'épaisseur de chaque feuillet de cuivre est supérieure à 30 micromètres, de préférence supérieure à 100 micromètres.

12. Dispositif (10) selon l'une quelconque des revendications précédentes, comprenant une couche intermédiaire (32, 132) de renforcement.

13. Dispositif (10) selon la revendication précédente, dans lequel la couche intermédiaire (32) comprend un PET transparent.

14. Dispositif (10) selon l'une quelconque des revendications précédentes, dans lequel les composants lumineux (22) émettent dans un spectre rouge de longueur d'onde comprise entre 620 et 640 nm.

15. Dispositif (10) selon l'une quelconque des revendications précédentes, dans lequel la surface du réseau inverse de pistes (27) d'isolation électrique représente moins de 10% de la surface de la couche de dissipation thermique (24) et de préférence moins de 5%, et au mieux de préférence moins de 2%.

## Patentansprüche

1. Flexible und verformbare Lichtvorrichtung (10), insbesondere dazu angepasst, mit einer zu behandelnden Hautoberfläche eines Benutzers in Kontakt zu kommen, von dem Typ umfassend:
• eine Lichtquelle (20, 120), die eine Vielzahl von Lichtemissionskomponenten (22) und ein elektrisches Verbindungsnetzwerk (24, 124) der Komponenten (22) untereinander für deren Stromversorgung umfasst,
• eine Wärmeableitschicht (24, 124) zur Ableitung der von der Lichtquelle (20) emittierten Wärme,
• ein verformbares Substrat (30), das eine Emissionsfläche des Lichtstroms begrenzt und die Lichtquelle (20) in seine Masse integriert, wobei die Wärmeableitschicht (24, 124) durch ein Nebeneinanderliegen von elektrisch und thermisch leitfähigen, verformbaren Metallplättchen (26, 26A, 26B) gebildet wird, wobei die Plättchen (26, 26A, 26B) voneinander getrennt und durch Zwischenräume (27) beabstandet sind, die ein inverses Netzwerk von elektrischen Isolationsbahnen der Metallplättchen (26, 26A, 26B) untereinander bilden, um durch Komplementarität das elektrische Verbindungsnetzwerk (24, 124) der Komponenten (22) untereinander in der Wärmeableitschicht (24, 124) zu definieren.

2. Vorrichtung (10) nach dem vorhergehenden Anspruch, umfassend erste (20) und zweite (120) Lichtquellen sowie erste (24) und zweite (124) Wärmeableitschichten, derart, dass die Vorrichtung (10) eine doppelseitige Lichtemissionsfläche aufweist.

3. Vorrichtung (10) nach einem der vorhergehenden Ansprüche, wobei jede Komponente (22) als Brücke zwischen zwei Plättchen (26, 26A, 26B) montiert ist.

4. Vorrichtung (10) nach einem der vorhergehenden Ansprüche, wobei die verformbaren Metallplättchen (26, 26A, 26B) flexible Zungen bilden, die eine Matrix aus im Wesentlichen parallelen Streifen darstellen, die nebeneinander angeordnet und durch die Zwischenräume (27) voneinander getrennt sind, und wobei die Komponenten (22) als Brücke zwischen zwei benachbarten Plättchen (26, 26A, 26B) montiert sind, wodurch eine Reihe von Komponenten (22) gebildet wird, die durch die zwei benachbarten Plättchen (26, 26A, 26B) elektrisch parallel geschaltet sind.

5. Vorrichtung (10) nach einem der vorhergehenden Ansprüche, wobei die Komponente (22) eine Leuchtdiode (LED) ist.

6. Vorrichtung (10) nach einem der vorhergehenden Ansprüche, umfassend mindestens ein seitliches Eckplättchen (26A, 26B) mit einer allgemeinen L-Form, das eine Endkontaktklemme umfasst, die einen Kontaktfleck zum elektrischen Anschluss an einen externen Stromversorgungsstecker (28) bildet.

7. Vorrichtung (10) nach einem der vorhergehenden Ansprüche, wobei das Metallplättchen (26) aus einem Material hergestellt ist, das im Wesentlichen Kupfer umfasst.

8. Vorrichtung (10) nach dem vorhergehenden Anspruch, wobei das Plättchen (26) eine allgemein polygonale Form aufweist, beispielsweise rechteckig, quadratisch oder L-förmig, und/oder mit abgerundeten Kanten.

9. Vorrichtung (10) nach einem der vorhergehenden Ansprüche, wobei die Schicht des Substrats (30) aus einem Material hergestellt ist, das im Wesentlichen Polyurethan umfasst.

10. Vorrichtung (10) nach einem der vorhergehenden Ansprüche, wobei jedes Plättchen (26) aus einer Kupferfolie gebildet ist.

11. Vorrichtung (10) nach dem vorhergehenden Anspruch, wobei die Dicke jeder Kupferfolie größer als 30 Mikrometer ist, vorzugsweise größer als 100 Mikrometer.

12. Vorrichtung (10) nach einem der vorhergehenden Ansprüche, umfassend eine dazwischenliegende Verstärkungsschicht (32, 132).

13. Vorrichtung (10) nach dem vorhergehenden Anspruch, wobei die Zwischenschicht (32) ein transparentes PET umfasst.

14. Vorrichtung (10) nach einem der vorhergehenden Ansprüche, wobei die Lichtkomponenten (22) in einem roten Spektrum mit einer Wellenlänge zwischen 620 und 640 nm emittieren.

15. Vorrichtung (10) nach einem der vorhergehenden Ansprüche, wobei die Fläche des inversen Netzwerks von elektrischen Isolationsbahnen (27) weniger als 10 % der Fläche der Wärmeableitschicht (24) ausmacht, vorzugsweise weniger als 5 % und idealerweise weniger als 2 %.

## Claims

1. A flexible and deformable light-emitting device (10), particularly adapted to come into contact with a surface of a user's skin to be treated, of the type comprising:
• a light source (20, 120) comprising a plurality of light-emitting components (22) and an electrical interconnection network (24, 124) for interconnecting the components (22) with each other for their electrical power supply,
• a heat dissipation layer (24, 124) for dissipating the heat emitted by the light source (20),
• a deformable substrate (30) delimiting a light flux emission face, incorporating the light source (20) within its mass, the heat dissipation layer (24, 124) being formed by a juxtaposition of electrically and thermally conductive deformable metallic plates (26, 26A, 26B), the plates (26, 26A, 26B) being disjointed and separated by gaps (27) forming an inverse network of electrical insulation tracks for insulating the metallic plates (26, 26A, 26B) from each other to define, by complementarity, the electrical interconnection network (24, 124) of the components (22) with each other within the heat dissipation layer (24, 124).

2. The device (10) according to the preceding claim, comprising first (20) and second (120) light sources and first (24) and second (124) heat dissipation layers such that the device (10) is a double-sided light-emitting device.

3. The device (10) according to any one of the preceding claims, wherein each component (22) is bridge-mounted between two plates (26, 26A, 26B).

4. The device (10) according to any one of the preceding claims, wherein the deformable metallic plates (26, 26A, 26B) form flexible tongues forming a matrix of substantially parallel strips, juxtaposed and separated from each other by the gaps (27), and the components (22) are bridge-mounted between two adjacent plates (26, 26A, 26B) forming a row of components (22) electrically connected in parallel by the two adjacent plates (26, 26A, 26B).

5. The device (10) according to any one of the preceding claims, wherein the component (22) is a light-emitting diode.

6. The device (10) according to any one of the preceding claims, comprising at least one lateral corner plate (26A, 26B) with a general L-shape comprising an end contact terminal forming a contact pad for electrical connection to an external power supply connector (28).

7. The device (10) according to any one of the preceding claims, wherein the metallic plate (26) is made of a material essentially comprising copper.

8. The device (10) according to the preceding claim, wherein the plate (26) has a generally polygonal shape, for example rectangular, square or "L-shaped", and/or with rounded edges.

9. The device (10) according to any one of the preceding claims, wherein the layer of the substrate (30) is made of a material essentially comprising polyurethane.

10. The device (10) according to any one of the preceding claims, wherein each plate (26) is formed of a copper foil.

11. The device (10) according to the preceding claim, wherein the thickness of each copper foil is greater than 30 micrometers, preferably greater than 100 micrometers.

12. The device (10) according to any one of the preceding claims, comprising an intermediate reinforcement layer (32, 132).

13. The device (10) according to the preceding claim, wherein the intermediate layer (32) comprises a transparent PET.

14. The device (10) according to any one of the preceding claims, wherein the light-emitting components (22) emit in a red spectrum with a wavelength between 620 and 640 nm.

15. The device (10) according to any one of the preceding claims, wherein the surface of the inverse network of electrical insulation tracks (27) represents less than 10% of the surface of the heat dissipation layer (24), and preferably less than 5%, and ideally less than 2%.
